Europäisches Patentamt

European Patent Office

Office européen des brevets

(11) Publication number: **0 267 611 B1**

# EUROPEAN PATENT SPECIFICATION

(45) Date of publication of patent specification: **26.05.93** (51) Int. Cl.5: **C12P 21/08**, C12N 15/00, A61K 39/395

(21) Application number: **87116724.3**

(22) Date of filing: **12.11.87**

(54) Antibody against interleukin-1.

(30) Priority: **13.11.86 JP 270992/86**

(43) Date of publication of application:
**18.05.88 Bulletin 88/20**

(45) Publication of the grant of the patent:
**26.05.93 Bulletin 93/21**

(84) Designated Contracting States:
**CH DE ES FR GB IT LI NL SE**

(56) References cited:
EP-A- 0 187 991
EP-A- 0 188 920
EP-A- 0 220 063

IMMUNOBIOLOGY, vol. 172, nos. 3-4-5, 1986, pages 346-356; T.A. LUGER et al.: "Monoclonal anti-IL 1 is directed against a common site of human IL 1alpha and 1L 1beta"

(73) Proprietor: **OTSUKA PHARMACEUTICAL CO., LTD.**
**9, Kandatsukasacho 2-chome**
**Chiyoda-ku Tokyo 101(JP)**

(72) Inventor: **Ohmoto, Yasukazu 35-6, Aza-Hachishimo**
**Sasakino Matsushige-cho**
**Itano-gun Tokushima-ken(JP)**
Inventor: **Nagamura, Kenji 8-7, Aza-Shichimai**
**Tateiwa Muya-cho**
**Naruto-shi Tokushima-ken(JP)**
Inventor: **Aoki, Hisakazu 8, Aza-Nibangoshi**
**Hiroshima Matsushige-cho**
**Itano-gun Tokushima-ken(JP)**
Inventor: **Hirai, Yoshikatsu 5-49, Aza-Ekogawa**
**Shinkirai Kitajima-cho**
**Itano-gun Tokushima-ken(JP)**

(74) Representative: **Hansen, Bernd, Dr.rer.nat. et al**
**Hoffmann, Eitle & Partner Patentanwälte Arabellastrasse 4 Postfach 81 04 20**
**W-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL EXP. MEDICA, vol. 163, February 1986, pages 463–468, The Rockefeller University Press; A. KOCK et al.: " Characterization of a monoclonal antibody directed against the biologically active site of human interleukin 1"

PROGRESS IN LEUKOCYTE BIOLOGY, vol. 2, 1985, pages 491–500, Alan R. Liss, Inc.; T.A. LUGER et al.: "Characterizarion of a monclonal antibody directed against human interleukin 1+

JOURNAL OF IMMUNOLOGY, vol 131, no. 4, October 1983, pages 1834–1837, The American Association of Immunologists, US; S.B. MIZEL et al.: "Preparation of goat antibodies against interleukin 1: use of an immunoadsorbent to purify interleukin 1"

JOURNAL OF IMMUNOLOGY, vol. 138, no. 6, March 1987, pages 1804–1812, The American Association of Immunologists; T KASAHARA et al.: "Preparation and characterization of polyclonal and monoclonal antibodies against human interleukin 1alpha (IL 1alpha)1"

JOURNAL OF IMMUNOLOGY, vol. 138, no. 12, June 1987, pages 4236–4242, The American Association of Immunologists, US; J.S. KENNEY et al.: "Monoclonal antibodies to human recombinant interleukin 1 (IL 1)beta: Ouantitation of Il 1Beta and inhibition of biological activity"

**Description**

The present invention relates to antibodies to Interleukin−1 (IL−1), and more particularly to novel antibodies to human IL−1 which make it possible to immunologically purify and determine IL−1.

Human IL−1 is produced by macrophage monocytes and many other cells and is known to exhibit various molecular forms and biological activities. Presently known as human IL−1's are two kinds which are different in isoelectric point, i.e., IL−1α and IL−1β. The primary structures of these IL−1's have been clarified. (Nature, 315, p641 (1985); J. Exp. Med., 164, p237 (1986); etc.)

Extensive research has been conducted on the application of IL−1's as drugs. Attention has been directed to the determination of IL−1's especially from clinical samples for the research on various immunodeficiency diseases and on abnormal immune responses and for the clinical diagnosis of these diseases and abnormalities.

At present, bioassays are known as techniques for determining IL−1's. With this method, IL−1 is determined as the biological activity of the test sample. This method nevertheless is inferior in the ease of procedure and accuracy and involves the necessity of always giving consideration to some component interfering with the measurment. Moreover, the method has the serious drawback of being unable to determine IL−1α and IL−1β as distinguished from each other since they exhibit the same activity.

An object of the present invention is to provide antibodies to human IL−1α and human IL−1β.

Another object of the invention is to provide antibodies to human IL−1's for use in a novel method of immunologically determining human IL−1α and IL−1β each individually.

Another object of the present invention is to provide antibodies to human IL−1's capable of neutralizing or suppressing the biological activity of IL−1 and useful in curing various diseases producing an abnormally large quantity of IL−1.

Another object of the invention is to provide a technique for preparing these antibodies.

These objects and other features of the invention will become apparent from the following detailed description.

The present invention provides a monoclonal antibody to human IL−1 which is characterized in that the antibody has specific reactivity with human IL−1α or human IL−1β, and a process for preparing the same.

The invention further provides a novel method of immunoassay by which human IL−1α and human IL−1β can be easily determined individually as distinguished from each other with high sensitivity and high accuracy, using the antibody of the invention.

Since the antibody of the invention is specific to human IL−1α or human IL−1β, the use of the antibody provides a specific method of purifying the IL−1, for example, by affinity chromatography.

Further, the antibody of the invention includes a type which can neutralize the biological activity of human IL−1 and which is useful for treating various diseases producing an abnormally large quantity of IL−1, such as chronic rheumatoid arthritis, thyroiditis, hepatitis, nephritis and like chronic inflammatory diseases; arteriosclerosis, kawasaki disease and like angitis; disseminated intravascular coagulation (DIC) syndrome; blood cancer, etc. The antibody can suppress the accelerated activity of the human IL−1 produced by the above diseases. Therefore the antibody of the invention provide drugs useful for preventing and curing these diseases.

The antibody of the present invention can be prepared by using human IL−1α or human IL−1β as an immunizing antigen. More specifically, the present antibody can be prepared, for example, by immunizing a mammal with the antigen, fusing the plasmacytes (immune cells) of the mammal with plasmacytoma cells of a mammal to obtain hybridoma cells, cloning the cells, selecting a clone which produces the desired antibody recognizing human IL−1α or IL−1β, and incubating the clone.

Human IL−1α or human IL−1β for use in the above process as the immunizing antigen is not limited specifically. Useful for this purpose are, for example, a culture supernatant containing human IL−1α and/or human IL−β derived by a known in vitro method or purified standard product thereof, human IL−1α or human IL−1β prepared by gene engineering techniques, and a synthetic polypeptide having a portion of the amino acid sequence of such human IL−1.

The mammal to be immunized with the antigen is not limited specifically. However, it is desirable to select a suitable animal in view of the amenability to the fusion of its cells with the plasmacytoma cell to be used. Generally, mice and rats are advantageously usable.

The mammal is immunized by a usual method, for example, by intravenously, subcutaneously or intraperitoneally giving the antigen, more specifically by administering the antigen to the animal several times every 2 to 14 days at a total dose of about 100 to 500 µg/animal. A usual adjuvant is usable for the administration when required. It is desirable that the immune cells to be used be spleen cells removed about 3 days after the final administration of the immunizing antigen.

3

Plasmacytoma cells of mammals as the other host cells to be fused with the immune cells are various known cell lines including myeloma cells, such as P3 (P3/X63 − Ag8) [Nature, 256, 495 − 497 (1975)], P3 − U1 [Current Topics in Microbiology and Immunology, 81, 1 − 7 (1978)], NS − 1 (Eur. J. Immunol., 6, 511 − 519 (1976)], MPC − 11 [Cell, 8, 405 − 415 (1976)], SP2/0 [Nature, 276, 269 − 270 (1978)], FO [J. Immunol. Meth., 35, 1 − 21 (1980)], X63.6.5.3. [J. Immunol., 123, 1548 − 1550 (1979)], S194 [J. Exp. Med., 148, 313 − 323 (1978)] etc., R210 of rat [Nature, 277, 131 − 133 (1979)].

The fusion between the immune cells and plasmacytoma cells is conducted basically by a known method, such as the method of Milstein et al (Method in Enzymology, Vol. 73, p3 (1981)). More specifically, the fusion reaction is conducted, for example, in a usual nutrient medium in the presence of a usual fusion promoting agent such as polyethylene glycol (PEG) or hemagglutinating virus of Japan (HVJ). To achieve an improved fusion efficiency, auxiliary agents such as dimethyl sulfoxide can be added to the medium when so desired. The immune cells and plasmacytoma cells are used in a usual ratio. For example, immune cells are used in about 1 to about 10 times the amount of plasmacytoma cells. Examples of media useful for the fusion are RPMI − 1640 medium and MEM medium which are usually used for proliferating plasmacytoma cells, and various other media which are used for incubating cells of this type. Generally it is desirable to use such media with the serum supplement, such as fetal calf serum (FCS), removed therefrom. To effect fusion, predetermined quantities of immune cells and plasmacytoma cells are thoroughly mixed together in the medium, and a solution of PEG having an average molecular weight of about 1000 to about 6000 is admixed, as preheated to about 37°C, with the medium usually at a concentration of about 30 to about 60 W/V%. Subsequently, a suitable medium is admixed with the culture from time to time, followed by centrifuging and removal of the supernatant every time. Repetition of this procedure gives the desired hybridoma.

The desired hybridoma obtained is separated by incubation in a usual selection medium such as HAT medium (containing hypoxanthine, aminopterin and thymidine). The incubation with the HAT medium is conducted for a period of time, usually several days to several weeks, which is sufficient to extinguish the cells (e.g. unfused cells) other than the desired hybridoma cells. The hybridoma cells obtained are then subjected to the usual limiting dilution method to retrieve the clones producing the desired antibody, followed by the production of monoclonal antibody.

The antibody − producing clones can be detected by various methods which are generally used for detecting antibodies, such as the ELISA method (Engvall, E., Meth. Enzymol., 70, 419 − 439 (1980), plaque method, spot method, agglutination reaction method, Ouchterlony method and radioimmunoassay (RIA) ("Hybridoma Method and Monoclonal Antibodies," published by R & D. Planning Co., Ltd., pp. 30 − 53, March 5, 1982). The immunizing antigen is usable for the detection.

The hybridoma thus obtained and producing the desired monoclonal antibody which recognizes human IL − 1α or human IL − 1β can be subcultured in a usual medium and preserved for a prolonged period of time in liquid nitrogen.

The desired antibody of the invention can be collected from the antibody − producing hybridoma as a culture supernatant by incubating the hybridoma in the usual manner, or as the ascites of a mammal which is amenable to the proliferation of the hybridoma, by administering the hybridoma to the animal. The former method is suitable for preparing the antibody with a high purity, while the latter method is suited to the quantity production of the antibody.

When desired, the antibody of the invention can be easily purified by a usual method such as salting out, gel filtration, affinity chromatography or the like.

The monoclonal antibody thus obtained according to the invention has reactivity specific to human IL − 1α or human IL − 1β.

The antibodies of the invention include those of the type acting to neutralize the biological activity of human IL − 1. Such antibodies are suitable for specifically determining human IL − 1 having biological activity. Such antibodies, particularly the antibodies capable of recognizing the IL − 1 receptor binding site of IL − 1 molecule are effective for curing various diseases which abnormally produce large quantity of IL − 1.

The antibodies of the invention further include those of the type which recognizes different sites of the human IL − 1 molecule, free of steric hindrance between antibodies and capable of binding to the human IL − 1 molecule. These antibodies are very useful for an immunoassay, for example, by the sandwich technique.

Additionally, the antibodies of the invention include those of the type having high reactivity especially in a liquid or solid phase system. These antibodies are very advantageously usable for liquid phase or solid phase immunoassays.

Among the antibodies of the invention, preferred examples are as follows.

1) An antibody having a recognition site in the sequence of amino acids Nos.121 to 140 of human IL − 1β.

2) An antibody having a recognition site in the sequence of amino acids Nos.24 to 82 of human IL − 1β.

3) An antibody having a recognition site in the sequence of amino acids Nos.83 to 102 of human IL − 1β.

4 An antibody having a recognition site in the sequence of amino acids Nos.145 to 148 of human IL − 1β.

The invention provides a monoclonal antibody which is specific to human IL − 1α or human IL − 1β. The use of this antibody provides a method of accurately determining with high sensitivity and high specificity human IL − 1α or human IL − 1β as contained, for example, in clinical samples in a low concentration, by an immunoassay.

The invention will be described in greater detail with reference to the following examples, which in no way limit the invention.

Example 1

Preparation of antibodies to human IL − 1β

Human IL − 1β (10 μg) obtained by gene recombination techniques (Biochemistry, 58, No.8, p.840 (1986); EPO No.187991) was intraperitoneally given to a BALB/C mouse along with complete Freund's adjuvant. The interleukin was further given every 3 to 4 weeks twice at the same dose along with an incomplete adjuvant to immunize the animal. Three to four weeks thereafter, a solution of 30 μg of human IL − 1β in physiological saline was intravenously given to the animal for final immunization. Three to four days thereafter, cell fusion was conducted in the usual manner (see, for example, Method in Enzymology, 73, p.3 (1981)), using spleen cells collected from the immunized animal and myeloma cells (P3U1, Current Topics in Microbiology and Immunology, 81, 1 − 7 (1978)) in the ratio of 10:1 and polyethylene glycol (PEG − 4000).

The desired hybridoma cells were selected on HAT medium. The supernatant was tested by an enzyme immunoassay using a 96 − well microplate coated with human IL − 1β and peroxidase − labeled goat anti − mouse globulin (product of E. Y. Lab.) to detect cells producing the desired antibodies to human IL − 1β.

The cells were repeatedly cloned by the limiting dilution method to obtain 7 clones producing the desired antibodies.

The characteristics of the antibody obtained from each of the clones were determined by the following methods.

(1) Subclass of antibody

Determined using a mouse antibody subclass detecting kit (product of Bio − Rad).

(2) Antibody production level

Expressed in terms of the amount of IgG (μg/ml) in the culture supernatant of hybridoma when the highest cell concentration was achieved.

(3) Potency

Determined by the following procedures.

RIA:

According to the Iodogen method (B.B.R.C., 80, 849 − 857 (1978)), 100 μl (about 10000 cpm) of $^{125}$I − labeled human IL − 1β, 100 μl of a dilution of hybridoma culture supernatant and 100 μl of PBS containing 0.01% NaN$_3$, 5mM EDTA and 0.1% BSA were mixed together and reacted at 4°C. To the reaction mixture were added 50 μl of normal goat serum serving as a carrier protein and 600 μl solution of 20% PEG in PBS, and the mixture was stirred, then allowed to stand in ice bath for 20 minutes and thereafter centrifuged. The radioactivity of the sediment was measured by a gamma counter. The dilution ratio of the culture supernatant resulting in the sedimentation of 15% of $^{125}$I − labeled IL − 1β was determined as RIA potency.

EIA:

Human IL−1$\beta$ adjusted to a concentration of 20 $\mu$g/ml was placed into the wells of a 96−well microplate (100 $\mu$l/well) and allowed to stand at room temperature for 1 hour. After washing the plate with PBS, BSA was applied to the plate for blocking nonspecific adsorption, and the plate was then washed with PBS−Tween 20. A dilution of hybridoma culture supernatant was placed into the well (100 $\mu$l/well), followed by reaction at 37°C for 2 hours and then by washing. Peroxidase−labeled anti−mouse immune globulin (product of Cappel Labs. Inc., X5000) was then placed into the wells in an amount of 100 $\mu$l/well, followed by reaction under the same conditions as above. After washing the plate, the enzyme activity on the plate was determined by colorimetric analysis. The dilution ratio of the culture supernatant resulting in $OD_{492} = 1.0$ was determined as EIA potency.

RIA/IgG and EIA/IgG:

These are the values obtained by dividing the RIA potency and EIA potency by the antibody production level.

RIA/IgG represents the reactivity of the antibody of the invention (with $^{125}$I−labeled human IL−1$\beta$) in the liquid phase system, while EIA/IgG stands for the reactivity of the present antibody (with human IL−1$\beta$ in the form of a solid phase) in the solid phase system.

(4) Molecular weight

The hybridoma was incubated intraperitoneally in a mouse, and the $IgG_1$ fraction collected and purified from the ascites using IgG purifying kit (MOPS Kit, product of Bio−Lad) was checked for molecular weight (which was the sum of the molecular weights of the heavy chain and the light chain as determined by SDS−PAGE).

(5) Cross reactivity

Determined by Western blotting (Proc. Natl. Acad. Sci., U.S.A., 76, 4350 (1979); Anal. Biochem., 112, 195 (1981)) using human recombinant proteins prepared by genetic engineering techniques. The symbol "−" represents absence of cross reactivity.

(6) Binding constant (kd)

Determined by Scatchard blot analysis.

(7) Neutralizing activity

Determined from LAF activity (J. Immunol., 116, 1466 (1976)). The mark "+" represents presence of neutralizing activity.

Table 1 shows the characteristics thus determined.

Table 1

| Antibody | Antibody Subclass | production level (μg/ml) | Potency | | | |
|---|---|---|---|---|---|---|
| | | | RIA | RIA/IgG | EIA | EIA/IgG |
| ANOC201 | IgG$_1$ | 59 | X3600 | 61 | X3200 | 54 |
| ANOC202 | IgG$_1$ | 127 | X3200 | 25 | X7000 | 55 |
| ANCO203 | IgG$_1$ | 100 | X5600 | 56 | X9800 | 98 |
| ANCO204 | IgG$_1$ | 200 | X2.1 | 1 | X15000 | 75 |
| ANCO205 | IgG$_1$ | 22 | X2100 | 95 | X2000 | 91 |
| ANCO206 | IgG$_1$ | 59 | X20 | 0.3 | X400 | 7 |
| ANCO207 | IgG$_1$ | 120 | X23000 | 191 | X8000 | 67 |

7

## Table 1 (continued)

| Molecular Antibody | weight (kd) | Cross reactivity | | | | Binding constant | | Neutralizing activity |
|---|---|---|---|---|---|---|---|---|
| | | IL-2 | IL-1α | GM-CSF | TNF | (kd) | (M/L) | |
| ANOC201 | 158 | − | − | − | − | 5.9 X $10^{-8}$ | | + |
| ANOC202 | 160 | − | − | − | − | 1.8 X $10^{-7}$ | | + |
| ANOC203 | 162 | − | − | − | − | 3.6 X $10^{-8}$ | | + |
| ANOC204 | 163 | − | − | − | − | − | | + |
| ANOC205 | 159 | − | − | − | − | 3.0 X $10^{-8}$ | | + |
| ANOC206 | 158 | − | − | − | − | − | | + |
| ANOC207 | 168 | − | − | − | − | 4.3 X $10^{-8}$ | | + |

(8) Determination of binding site

The following fragments of human IL−1β were prepared by the method described in EPO No.187991 for preparing human IL−1β.

Fragments

| | |
|---|---|
| N − 10 : | Polypeptide of human IL − 1β comprising amino acids Nos.11 − 153 |
| N − 16 : | Polypeptide of human IL − 1β comprising amino acids Nos.17 − 153 |
| N − 23 : | Polypeptide of human IL − 1β comprising amino acids Nos.24 − 153 |
| C − 82 : | Polypeptide of human IL − 1β comprising amino acids Nos.1 − 82 |
| C − 102: | Polypeptide of human IL − 1β comprising amino acids Nos.1 − 102 |
| C − 120: | Polypeptide of human IL − 1β comprising amino acids Nos.1 − 120 |
| C − 140: | Polypeptide of human IL − 1β comprising amino acids Nos.1 − 140 |
| C − 144: | Polypeptide of human IL − 1β comprising amino acids Nos.1 − 144 |
| C − 148: | Polypeptide of human IL − 1β comprising amino acids Nos.1 − 148 |

To E. coli cells expressing human IL − 1β or each of the fragments was added 100 μl of 6.25 mM Tris buffer (pH 6.8, containing 2% SDS, 5% 2ME, 10% glycerol and 0.001% BPB) to obtain a solution, which was then boiled at 100°C for 2 minutes to obtain a sample for electrophoresis. The samples thus prepared were tested for reactivity with antibodies of the invention by Western blotting.

Table 2 below shows the result.

Table 2

| Human IL-1β | ANOC201 | ANOC202 | ANOC203 | ANOC204 | ANOC205 | ANOC206 | ANOC207 |
|---|---|---|---|---|---|---|---|
| 1-153 | + | + | + | + | + | + | + |
| 11-153 | + | + | + | + | + | + | + |
| 17-153 | + | + | + | + | + | + | + |
| 24-153 | + | + | + | + | + | + | + |
| 1- 82 | - | + | - | + | - | - | - |
| 1-102 | - | + | + | + | - | - | - |
| 1-120 | - | + | + | + | - | - | - |
| 1-140 | + | + | + | + | - | + | + |
| 1-144 | + | + | + | + | - | + | + |
| 1-148 | + | + | + | + | + | + | + |
| Binding site | 121-140 | 24-82 | 83-102 | 24-82 | 145-148 | 121-140 | 121-140 |

Table 2 reveals the following. Antibodies ANOC 201, 206 and 207 each have a recognition site in the sequence of amino acids Nos.121 to 140 of human IL−1β, antibodies ANOC 202 and 204 in the sequence of amino acids Nos.24 to 82, antibody ANOC 203 in the sequence of amino acids Nos.83 to 102, and antibody ANOC 205 in the sequence of amino acids Nos.145 to 148.

(9) Steric hindrance test of present antibodies

Each of the antibodies of the invention was labeled with $^{125}$I by the Iodogen method to obtain a labeled antibody.

Each of the present antibody was caused to be adsorbed by a polystyrene bead (6.4 mm in diameter) by the physical adsorption method (Clin. Chem. Acta., 135, 263 (1983)) to obtain an insolubilized antibody.

The insolubilized antibody was reacted with 20 ng of human IL−1β in 0.5 ml of PBS solution containing 1% BSA and 0.01% thimerosal at 37°C for 2 hours. The same PBS solution (0.5 ml) as above containing about 100000 cpm of the labeled antibody obtained above was added to the bead which was washed after the reaction, followed by reaction at 37°C for 2 hours with shaking.

After washing the bead, the radioactivity bound to the bead was counted to determine the reactivity between the antibodies (whether or not the sandwich of insolubilized antibody − human IL−1β − labeled antibody was formed).

Consequently, no sandwich was formed between antibodies ANOC 201, 206 and 207, indicating that these antibodies recognize the substantially the same site of IL−1β.

No hindrance was found in the reaction between all the other antibodies tested (except in the reaction the antibody with itself).

(10) Inhibition of binding of IL−1β to IL−1 receptor on fibroblast (Test A)

BALB/c3T3 fibroblasts (ATCC CCL−163) almost uniformly grown over a 6−well plate ($1 \times 10^6$ cells/well) were reacted at 37°C for 4 hours with 8500 cpm/well of $^{125}$I−labeled IL−1β (at least 250 $\mu$Ci/$\mu$g protein in specific activity, prepared by the method of Bolton and Hunter (Biochem. J., 133, 529 (1973)) and with an antibody of the invention (anti−IL−1β monoclonal antibody) preincubated at 37°C in D−MEM supplemented with 10% FCS. Subsequently, 50 $\mu$l of rat serum and 250 $\mu$l of 20% PEG were reacted with the reaction mixture at 4°C for 20 minutes. The plate was then centrifuged (12000 r.p.m., 15 minutes) to obtain a sediment (bound product) and a supernatant (unbound product) separated therefrom. The bound reactivity was then measured by a gamma counter.

The activity (%) of the present antibody to inhibit IL−1β from binding to the IL−receptor on the fibroblast was calculated from the following equation.

$$\text{Inhibition activity (\%)} = \frac{A - C}{A - B} \times 100$$

wherein
   A:   radioactivity of control without the antibody of invention
   B:   radioactivity nonspecifically adsorbed by the plate
   C:   Radioactivity due to the use of the present antibody
   In this test, the value A was 3895 cpm/well, and the value C was 301 cpm.

Table 3 shows the results achieved by antibodies of the invention, i.e. ANOC 203 and 205.

## Table 3

| Concn. of antibody (μg/ml) | Inhibition activity | |
| --- | --- | --- |
| | ANOC203 | ANOC205 |
| 0 | 100 | 100 |
| 6.3 | 24 | 98 |
| 12.5 | 16 | 105 |
| 25 | 7 | 95 |
| 50 | 8 | 103 |
| 100 | 2 | 99 |

Table 3 shows that ANOC 203 of the invention inhibits $^{125}I$–labeled IL–1$\beta$ from binding to the IL–1 receptor, indicating that the antibody recognizes the IL–1 receptor binding site of IL–1$\beta$. Antibody ANOC 205 of the invention has substantially no inhibition activity. This indicates that the antibody recognizes a site different from the IL–1 receptor binding site.

(11) Inhibition of binding to IL–1 receptor on fibroblast (Test B)

Into each of wells having fibroblasts grown therein were placed $^{125}I$–labeled IL–1$\alpha$, 10 ng/ml of IL–1$\beta$ and a specified quantity of an antibody of the invention, and the mixture was reacted in the same manner as in the procedure (10) above. After similarly centrifuging the plate, the radioactivity of the well was measured, and the activity (%) of the present antibody to inhibit IL–1$\beta$ from binding to the IL–1 receptor on the fibroblast was calculated.

In this test, the value A was 8278 cpm/well, and the value C was 410 cpm.

Table 4 shows the results achieved by antibodies of the invention, i.e. ANOC 203 and 205.

## Table 4

| Antibody concn. (μg/ml) | IL-1β concn. (ng/ml) | Inhibition activity | |
| --- | --- | --- | --- |
| | | ANOC203 | ANOC205 |
| 0 | 0 | 100 | 100 |
| 0 | 10 | 13.2 | 13.2 |
| 10 | 10 | 24.4 | 14.8 |
| 100 | 10 | 58.6 | 15.0 |

Table 4 reveals the following. IL–1$\beta$ inhibits $^{125}I$–labeled IL–1$\alpha$ from binding to the IL–1 receptor, whereas the present antibody ANOC 203 recognizes the site where IL–1$\beta$ binds to the IL–1 receptor, inhibiting IL–1$\beta$ from binding to the receptor and thus exhibiting recovered binding inhibitory activity. The present antibody ANOC 205 does not recognize the IL–1 receptor binding site for IL–1$\beta$, apparently failing to exhibit recovered binding inhibitory activity.

Example 2

Preparation of antibody to human IL − 1α

Clones producing the desired antibody to human IL − 1α were obtained in the same manner as in Example 1 using as an immunizing antigen human IL − 1α prepared in the same manner as human IL − 1β used above (Nature, 315, p.641 (1985)). ANOC 301, an antibody of the invention against IL − 1α, was obtained from the clones. This antibody had the following characteristics.

```
Subclass                    : IgG₂ₐ

Antibody production level   : 75 µg/ml

Potency                     : RIA   X3200

                              EIA   X2000

Cross reactivity            : No cross reactivity on IL-2,

                              IL-1β, GM-CSF and TNF

Binding constant (kd)       : 3.6 x 10⁻⁸ M/L

Neutralizing activity       : Active
```

Each of four hybrid cell lines obtained in the examples has been deposited in Fermentation Research Institute, Agency of Industrial Science and Technology since November 5, 1987 under the following deposition names and numbers, respectively.

| | | Name | Number |
|---|---|---|---|
| (1) | Hybrid cell line producing ANOC203 | KOCO203 | FERM BP-1551 |
| (2) | Hybrid cell line producing ANOC205 | KOCO205 | FERM BP-1552 |
| (3) | Hybrid cell line producing ANOC206 | KOCO206 | FERM BP-1553 |
| (4) | Hybrid cell line producing ANOC301 | KOCO301 | FERM BP-1554 |

**Claims**
**Claims for the following Contracting States : CH, DE, FR, GB, IT, LI, NL, SE**

1.  A monoclonal antibody against human interleukin − 1 characterized in that the antibody has specific reactivity with human interleukin − 1α or human interleukin − 1β.

2.  An antibody as defined in claim 1 which has specific reactivity with human interleukin − 1α.

3. An antibody as defined in claim 1 which has specific reactivity with human interleukin $-1\beta$.

4. An antibody as defined in claim 1 which possesses an activity to neutralize the biological activity of human interleukin $-1$.

5. An antibody as defined in claim 1 which has a recognition site in the sequence of amino acid Nos.121 to 140 of human interleukin $-1\beta$.

6. An antibody as defined in claim 1 which has a recognition site in the sequence of amino acid Nos.24 to 82 of human interleukin $-1\beta$.

7. An antibody as defined in claim 1 which has a recognition site in the sequence of amino acid Nos.83 to 102 of human interleukin $-1\beta$.

8. An antibody as defined in claim 1 which has a recognition site in the sequence of amino acid Nos.145 to 148 of human interleukin $-1\beta$.

9. An antibody as defined in claim 1 which is produced by the hybrid cell line FERM BP $-1551$.

10. An antibody as defined in claim 1 which is produced by the hybrid cell line FERM BP $-1552$.

11. An antibody as defined in claim 1 which is produced by the hybrid cell line FERM BP $-1553$.

12. An antibody as defined in claim 1 which is produced by the hybrid cell line FERM BP $-1554$.

13. The use of an antibody according to claims 1 to 12 for the preparation of a drug which is useful for treating a disease producing an abnormally large quantity of IL $-1$.

**Claims for the following Contracting State : ES**

1. A process for preparing a monoclonal antibody having specific reactivity with human interleukin $-1\alpha$ or human interleukin $-1\beta$, characterized by immunizing a mammal with human interleukin $-1\alpha$ or human interleukin $-1\beta$ serving as an immunizing antigen, fusing the immune cells with plasmacytoma cells of a mammal to obtain hybridoma cells, cloning the cells, selecting a clone which produces the desired antibody recognizing human interleukin $-1\alpha$ or human interleukin $-1\beta$, and incubating the clone.

2. The process according to claim 1, characterized in that the monoclonal antibody possesses an activity to neutralize the biological activity of human interleukin $-1$.

3. The process according to claim 1, characterized in that the antibody has a recognition site in the sequence of amino acid Nos. 121 to 140 of humun interleukin $-1\beta$.

4. The process according to claim 1, characterized in that the antibody has a recognition site in the sequence of amino acid Nos. 24 to 82 of human interleukin $-1\beta$.

5. The process according to claim 1, characterized in that the antibody has a recognition site in the sequence of amino acid Nos. 83 to 102 of human interleukin $-1\beta$.

6. The process according to claim 1, characterized in that the antibody has a recognition site in the sequence of amino acid Nos. 145 to 148 of human interleukin $-1\beta$.

7. The process according to claim 1, characterized in that the antibody is produced by the hybrid cell line FERM BP $-1551$.

8. The process according to claim 1, characterized in that the antibody is produced by the hybrid cell line FERM BP $-1552$.

14

9. The process according to claim 1, characterized in that the antibody is produced by the hybrid cell line FERM BP – 1553.

10. The process according to claim 1, characterized in that the antibody is produced by the hybrid cell line FERM BP – 1554.

**Patentansprüche**
**Patentansprüche für folgende Vertragsstaaten : CH, DE, FR, GB, IT, LI, NL, SE**

1. Monoklonaler Antikörper gegen Human – Interleukin – 1, dadurch gekennzeichnet, daß der Antikörper eine spezifische Reaktionsfähigkeit mit Human – Interleukin – 1$\alpha$ oder Human – Interleukin – 1$\beta$ aufweist.

2. Antikörper nach Anspruch 1, der eine spezifische Reaktionsfähigkeit mit Human – Interleukin – 1$\alpha$ aufweist.

3. Antikörper nach Anspruch 1, der eine spezifische Reaktionsfähigkeit mit Human – Interleukin – 1$\beta$ aufweist.

4. Antikörper nach Anspruch 1, der eine Aktivität aufweist, die biologische Aktivität von Human – Interleukin – 1 zu neutralisieren.

5. Antikörper nach Anspruch 1, der eine Erkennungsstelle in der Sequenz der Aminosäure Nr. 121 bis 140 von Human – Interleukin – 1$\beta$ hat.

6. Antikörper nach Anspruch 1, der eine Erkennungsstelle in der Sequenz der Aminosäure Nr. 24 bis 82 von Human – Interleukin – 1$\beta$ hat.

7. Antikörper nach Anspruch 1, der eine Erkennungsstelle in der Sequenz der Aminosäure Nr. 83 bis 102 von Human – Interleukin – 1$\beta$ hat.

8. Antikörper nach Anspruch 1, der eine Erkennungsstelle in der Sequenz der Aminosäure Nr. 145 bis 148 von Human – Interleukin – 1$\beta$ hat.

9. Antikörper nach Anspruch 1, der durch die Hybrid – Zellinie FERM BP – 1551 produziert wird.

10. Antikörper nach Anspruch 1, der durch die Hybrid – Zellinie FERM BP – 1552 produziert wird.

11. Antikörper nach Anspruch 1, der durch die Hybrid – Zellinie FERM BP – 1553 produziert wird.

12. Antikörper nach Anspruch 1, der durch die Hybrid – Zellinie FERM BP – 1554 produziert wird.

13. Verwendung eines Antikörpers nach den Ansprüchen 1 bis 12 zur Herstellung eines Arzneimittels, das zur Behandlung einer Krankheit verwendbar ist, die eine abnormal große Menge an IL – 1 produziert.

**Patentansprüche für folgenden Vertragsstaat : ES**

1. Verfahren zur Herstellung eines monoklonalen Antikörpers mit spezifischer Reaktionsfähigkeit mit Human – Interleukin – 1$\alpha$ oder Human – Interleukin – 1$\beta$, gekennzeichnet durch Immunisierung eines Säugers mit Human – Interleukin – 1$\alpha$ oder Human – Interleukin – 1$\beta$, das als ein Immunisierungsantigen dient, Verschmelzen der Immunzellen mit Plasmacytomzellen eines Säugers, um Hybridomzellen zu erhalten, Klonieren der Zellen, Selektieren eines Klones, der den gewünschten Antikörper produziert, der Human – Interleukin – 1$\alpha$ oder Human – Interleukin – 1$\beta$ erkennt, und Inkubieren des Klones.

2. Verfahren nach Anspruch 1, dadurch gekennzeichnet, daß der monoklonale Antikörper eine Aktivität besitzt, die biologische Aktivität von Human – Interleukin – 1 zu neutralisieren.

3. Verfahren nach Anspruch 1, dadurch gekennzeichnet, daß der Antikörper eine Erkennungsstelle in der Sequenz der Aminosäure Nr. 121 bis 140 von Human – Interleukin – 1$\beta$ hat.

4. Verfahren nach Anspruch 1, dadurch gekennzeichnet, daß der Antikörper eine Erkennungsstelle der Sequenz der Aminosäure Nr. 24 bis 82 von Human − Interleukin − 1$\beta$ hat.

5. Verfahren nach Anspruch 1, dadurch gekennzeichnet, daß der Antikörper eine Erkennungsstelle in der Sequenz der Aminosäure Nr. 83 bis 102 von Human − Interleukin − 1$\beta$ hat.

6. Verfahren nach Anspruch 1, dadurch gekennzeichnet, daß der Antikörper eine Erkennungsstelle in der Sequenz Aminosäure Nr. 145 bis 148 von Human − Interleukin − 1$\beta$ hat.

7. Verfahren nach Anspruch 1, dadurch gekennzeichnet, daß der Antikörper durch die Hybridzellinie FERM BP − 1551 produziert wird.

8. Verfahren nach Anspruch 1, dadurch gekennzeichnet, daß der Antikörper durch Hybridzellinie FERM BP − 1552 produziert wird.

9. Verfahren nach Anspruch 1, dadurch gekennzeichnet, daß der Antikörper durch die Hybridzellinie FERM BP − 1553 produziert wird.

10. Verfahren nach Anspruch 1, dadurch gekennzeichnet, daß der Antikörper durch die Hybridzellinie FERM BP − 1554 produziert wird.

**Revendications**
**Revendications pour les Etats contractants suivants : CH, DE, FR, GB, IT, LI, NL, SE**

1. Anticorps monoclonal dirigé contre l'interleukine − 1 humaine, caractérisé en ce que l'anticorps a une réactivité spécifique avec l'interleukine − 1$\alpha$ humaine ou l'interleukine − 1$\beta$ humaine.

2. Anticorps suivant la revendication 1, qui a une réactivité spécifique avec l'interleukine − 1$\alpha$ humaine.

3. Anticorps suivant la revendication 1, qui a une réactivité spécifique avec l'interleukine − 1$\beta$ humaine.

4. Anticorps suivant la revendication 1, qui possède une activité pour neutraliser l'activité biologique de l'interleukine − 1 humaine.

5. Anticorps suivant la revendication 1, qui a un site de reconnaissance dans la séquence des acides aminés No 121 à 140 de l'interleukine − 1$\beta$ humaine.

6. Anticorps suivant la revendication 1, qui a un site de reconnaissance dans la séquence des acides aminés No 24 à 82 de l'interleukine − 1$\beta$ humaine.

7. Anticorps suivant la revendication 1, qui a un site de reconnaissance dans la séquence des acides aminés No 83 à 102 de l'interleukine − 1$\beta$ humaine.

8. Anticorps suivant la revendication 1, qui a un site de reconnaissance dans la séquence des acides aminés No 145 à 148 de l'interleukine − 1$\beta$ humaine.

9. Anticorps suivant la revendication 1, qui est produit par la lignée cellulaire humaine FERM BP − 1551.

10. Anticorps suivant la revendication 1, qui est produit par la lignée cellulaire humaine FERM BP − 1552.

11. Anticorps suivant la revendication 1, qui est produit par la lignée cellulaire humaine FERM BP − 1553.

12. Anticorps suivant la revendication 1, qui est produit par la lignée cellulaire humaine FERM BP − 1554.

13. Utilisation d'un anticorps suivant les revendications 1 à 12 pour la préparation d'un médicament qui est utile pour traiter une maladie produisant une quantité anormalement élevée de IL − 1.

**Revendications pour l'Etat contractant suivant : ES**

1. Procédé pour préparer un anticorps monoclonal ayant une réactivité spécifique avec l'interleukine – 1$\alpha$ humaine ou l'interleukine – 1$\beta$ humaine, caractérisé par l'immunisation d'un mammifère avec l'interleukine – 1$\alpha$ humaine ou l'interleukine – 1$\beta$ humaine servant d'antigène immunisant, la fusion des cellules immunes avec des cellules de plasmocytome d'un mammifère pour obtenir des cellules d'hybridome, le clonage des cellules, la sélection d'un clone qui produit l'anticorps désiré reconnais – sant l'interleukine – 1$\alpha$ humaine ou l'interleukine – 1$\beta$ humaine, et l'incubation du clone.

2. Procédé suivant la revendication 1, caractérisé en ce que l'anticorps monoclonal possède une activité pour neutraliser l'activité biologique de l'interleukine – 1 humaine.

3. Procédé suivant la revendication 1, caractérisé en ce que l'anticorps a un site de reconnaissance dans la séquence des acides aminés No 121 à 140 de l'interleukine – 1$\beta$ humaine.

4. Procédé suivant la revendication 1, caractérisé en ce que l'anticorps a un site de reconnaissance dans la séquence des acides aminés No 24 à 82 de l'interleukine – 1$\beta$ humaine.

5. Procédé suivant la revendication 1, caractérisé en ce que l'anticorps a un site de reconnaissance dans la séquence des acides aminés No 83 à 102 de l'interleukine – 1$\beta$ humaine.

6. Procédé suivant la revendication 1, caractérisé en ce que l'anticorps a un site de reconnaissance dans la séquence des acides aminés No 145 à 148 de l'interleukine – 1$\beta$ humaine.

7. Procédé suivant la revendication 1, caractérisé en ce que l'anticorps est produit par la lignée cellulaire humaine FERM BP – 1551.

8. Procédé suivant la revendication 1, caractérisé en ce que l'anticorps est produit par la lignée cellulaire humaine FERM BP – 1552.

9. Procédé suivant la revendication 1, caractérisé en ce que l'anticorps est produit par la lignée cellulaire humaine FERM BP – 1553.

10. Procédé suivant la revendication 1, caractérisé en ce que l'anticorps est produit par la lignée cellulaire humaine FERM BP – 1554.